# EUROPEAN PATENT APPLICATION

(11) **EP 3 178 940 A1**
(43) Date of publication of application: **14.06.2017**
(21) Application number: 15198300.4
(22) Date of filing: 07.12.2015
(51) Int. Cl.: C12Q 1/68

(54) **DIAGNOSIS METHOD OF PANCREATIC CANCER**

(71) Applicant: Universite d'Aix Marseille, 13284 Marseille Cedex 07 (FR); INSTITUT NATIONAL DE LA SANTE ET DE LA RECHERCHE MEDICALE (INSERM), 75013 Paris (FR)
(72) Inventor: CRENON-CONSTARATAS, Isabelle, 13008 MARSEILLE (FR); MARTINEZ, Emmanuelle, 13610 Le Puy Sainte Réparade (FR); SILVY, Françoise, 13005 MARSEILLE (FR); MAS, Eric, 13010 MARSEILLE (FR); OUAISSI, Mehdi, 13009 MARSEILLE (FR); LOMBARDO, Dominique, 13011 MARSEILLE (FR)
(74) Representative: Gevers & Orès

(57) **Abstract**

The present invention relates to an *in vitro* method for the prognosis and/or diagnosis of a pancreatic cancer comprising the detection of a *BSDL* gene with an apparent cytosine insertion in a patient sample.

## Description

The present invention relates to the field of diagnosis methods for pancreatic cancer. In particular, the present invention concerns the *in vitro* prognosis/diagnosis of pancreatic cancer, especially of pancreatic adenocarcinoma, by detecting a BSDL transcript with an apparent cytosine insertion in a patient biological sample.

### BACKGROUND

With more than 277,000 new cases per year worldwide, pancreatic cancers represent 10% of all digestive cancers; 90% of these are pancreatic adenocarcinoma (PDAC). Pancreatic adenocarcinomas, as well as precancerous lesions, occur as a result of alterations affecting genes necessary to maintain cellular functions. These changes can include mutations, deletions or epigenetic modifications, and result in a gain or loss of function of genes. Alterations can be inherited (familial cancers, predispositions) or acquired, but in either case each stage of disease is associated with one or more types of alteration.

Patients with pancreatic cancer typically present with a lack of specific symptoms, leading to a delay in diagnosis. The survival rate is thus dramatically low with a case-fatality ratio of about 0.9. PDAC could be the second cause of death by cancer by the year 2030 and today has a 5-year survival rate of less than 4% in western countries. Its poor prognosis is mainly due to a lack of response to currently available therapies and to a very low curative resection rate (15% of patients) due to nonspecific symptoms, a lack of early biological markers, delayed diagnosis and metastasis formation.

Human bile salt-dependent lipase (BSDL), or carboxyl ester lipase (CEL), is mainly expressed by the acinar cells of the pancreas [1] and the lactating mammary gland [2]. The *BSDL* gene is located on band 34.3 of the long arm of chromosome 9 [3, 4]. The gene consists of 11 exons spanning 9884 base pairs (bp). The N-terminal domain of the protein includes bile salt-binding, catalytic, and N-glycosylation sites. This domain is encoded for by exons 1 to 10 and is well conserved across evolution [1], whereas exon 11 encodes the C-terminal domain with a region consisting in a variable number of tandem repeats (VNTR). Each repeat consists of 33 bp coding for 11 amino acids [5] with O-glycosylation sites [6], conferring a mucin-like structure to the C-terminal VNTR of BSDL [7]. The six most C-terminal amino-acid residues of BSDL are highly conserved and may play a role in enzyme activity or stability [8]. VNTR can vary slightly in sequence and the total number of repeats differs between alleles. However, VNTR on exon 11 can range from three to twenty-one repeats, leading to a high level of heterogeneity in protein size both within and between individuals [9, 10]. The presence of short forms of BSDL in human pancreatic juice has been reported by Meyer [16], and Duang and Borgström [17]. Furthermore in 2000, Pasqualini et al. showed that MiaPaCa-2 cells (cells from human pancreatic carcinoma) expressed an immunoreactive form of BSDL of approximately 70 kDa, which corresponds to the 1.8 kb cDNA obtained by RT-PCR using a pair of primers covering the full-length mRNA encoding the human BSDL [18]. However, the sequence of the transcript of 1.8 kb obtained from MiaPaCa-2 cells was shown to differ from that of the human pancreatic *BSDL* cDNA (2.2 kb) within the 3'-end region, which encodes mucin-like VNTR sequences [5]. The amino-acid sequence deduced from this 1.8 kb RT-PCR product was homologous with that of BSDL, except for a deletion of 110 amino acids occurring within VNTR. A recent publication demonstrated that characterization of mutations within a GC-rich domain of the genome requires a careful examination of electropherograms [19].

in light of the above, the inventors have hypothesized that the 1.8 kb transcript observed more than a decade ago in pancreatic tumor cells, might result from a modification of the *BSDL* gene sequence, overlooked due to the GC-richness within the VNTR. To confirm this hypothesis, they have characterized the sequence modifications in the C-terminal domain of BSDL occurring in pancreatic pathologies, in part pancreatic ductal adenocarcinoma. They have now showed that an apparent insertion of a cytosine residue in the *BSDL* (or *CEL*) gene can be associated with P DAC.

In the context of a clear need for diagnosis markers of pancreatic cancer, the identification of a reliable biomarker for the early diagnosis of pancreatic cancer is of great interest.

### DETAINED DESCRIPTION OF THE INVENTION

The present invention concerns an *in vitro* method for the diagnosis of a pancreatic cancer comprising the detection of a *BSDL* gene with an apparent cytosine insertion in a patient sample.

As illustrated in the examples provided in the experimental part, the inventors have demonstrated that the pancreatic tissue from patients with PDAC may exhibit, in addition to the wild-type transcript of *BSDL* gene, a transcript containing a cytosine insertion in the exon 11 of the *BSDL* gene, especially in at least one of the variable number of tandem repeats (VNTR) regions. This insertional mutation can be located in any of the VNTR. In the present study, such insertions were identified into VNTR 4, 5, 7 and 9 in either pancreatic samples or from the SOJ-6 pancreatic cell line issued from patients with PDAC. However, any position of this apparent insertion leads to a reading frame shift with a premature stop codon. This apparent insertion may result from a true insertion of a cytosine residue or from the deletion of some repeated sequences leaving a cytosine residue in place. In any case, this apparent cytosine insertion generates a frame shift with a new carboxy-terminal (Ct) sequence of the resulting truncated BSDL. The length of the protein can vary depending on the site of the insertion.

This shorten BSDL protein harbors a particular Ct sequence. Whereas the wild-type protein ends with a PAVIRF [Pro-Ala-Val-Ile-Arg-Phe] sequence, the truncated protein ends with a PRAAHG [Pro-Arg- Ala-Ala-His-Gly] sequence.

Accordingly, as used in the context of the invention, a *"BSDL* gene with an apparent cytosine insertion" corresponds (i) to a transcript of a *BSDL* gene having an apparent cytosine insertion into exon 11, more particularly in at least one VNTR, or (ii) to a truncated BSDL protein harboring a PRAAHG sequence at its Ct-extremity.

The data provided in the experimental part support the fact that the detection of the insertional mutation or of the related truncated protein can be biomarker for the early diagnosis of pancreatic cancer, in particular of stage 1 Pancreatic Intraepithelial Neoplasia (PanIN). Thus, the present invention consists in a method of early diagnosis of patients with a pancreatic cancer or suspected to develop such disease.

In a first embodiment, the method of prognosis and/or diagnosis of a pancreatic cancer of the invention relies on the detection of at least one cytosine insertion into at least one VNTR located in exon 11 of the *BSDL* gene.

In a particular embodiment, the cytosine insertion occurs in VNTR 4, 5, 7 or 9, more particularly in VNTR 4, 5 or 9.

The number of modified VNTR by cytosine insertions can be 1, 2 or more, so that the method of diagnosis is based on the fact that at least one cytosine insertion is detected. In a particular embodiment, the method of diagnosis of the invention consists in detecting one cytosine insertion. The cytosine insertion occurs in at least one VNTR, meaning it can occur in 1, 2 or more VNTR.

The detection of a cytosine insertion can be performed using any conventional method known by a skilled person in the art.

Detection can be done directly on the genomic DNA, for example by PCR (Polymerase Chain Reaction) or southern blot using DNA probe. Detection can also be performed on mRNA transcripts, for example using RT-PCR method, a well-known method for amplification of mRNA in a quantitative or semi-quantitative manner. Micro-array analysis may be appropriate if diagnosis is based on the combination of different biomarkers. For all these methods, the person skilled in the art will have to define the appropriate acid nucleic sequence that will allow to detect the target mutation.

In case of amplification of the genomic DNA by PCR, defining appropriate primers is routinely done. As an example, appropriate primers for detecting an insertional mutation into a VNTR of exon 11 of the *BSDL* gene can be those used in Table 1. Such primers are defined as follows:
Forward primer: 5' TCTTCTCACTCTGCAGGGACC 3' corresponds to SEQ ID NO.1
Reverse primer: 5' CAGGGGTATGAGGCTTTATTCA 3' corresponds to SEQ ID NO.2

In case of amplification of the mRNA by RT-PCR, appropriate primers can be defined as follows:
Forward primer: 5' GAACCAACTTCCTGCGCTAC 3' corresponds to SEQ ID NO.3
Reverse primer: 5' CAGGGGTATGAGGCTTTATTCA 3' corresponds to SEQ ID NO.2

The wild-type BSDL mRNA sequence corresponds to the SEQ ID NO.4 (NM_001807.4).

Methods directed toward the detection of an insertional mutation whether at genomic DNA or at mRNA level can be performed on a sample of pancreatic tissue. However, requiring access to pancreatic sample in the context of diagnosis is not appropriate. Starting from a more accessible DNA material is desirable.

A method of diagnosis of pancreatic cancer based on analysis of circulating tumoral DNA would be more convenient. Circulating tumoral DNA can be purified from blood before analysis. In a preferred embodiment, the nucleic acid are purified before analysis by removing the nucleated and anucleated cell population from the blood sample. The method of diagnosis of the invention can in particular be performed on blood serum. Sensitive methods such as q-PCR, dd-PCR, snap-shot ... using specific dedicated probes (LNA or others) could be appropriate for the detection of genomic DNA with insertional mutation in circulating tumoral DNA.

In particular embodiment, the present invention concerns an *in vitro* method for prognosis and/or diagnosis of a pancreatic cancer comprising the steps of (i) providing a patient sample, (ii) amplifying the VNTR regions of the exon 11 of the *BSDL* gene or the corresponding sequences in mRNA and (iii) detecting the presence of a cytosine insertion in the said gene. This method can further comprises the steps of administrating a treatment of pancreatic cancer when a cytosine insertion is present in at least one of said VNTR regions.

In another particular embodiment, the present invention concerns a kit for prognosis and/or diagnosis of a pancreatic cancer, based on the detection of a cytosine insertion into a VNTR motif located in exon 11 of the *BSDL* gene in a patient biological sample. Such kit can contains all the reagents necessary for the detection including in particular the probe or primers able to specifically match with the insertional mutation. In a particular embodiment such kit comprises specific primers allowing the amplification of the VNTR regions of the exon 11 of the *BSDL* gene or the corresponding sequences in mRNA present in a patient sample.

The invention also concerns a method of prognosis and/or diagnosis of a pancreatic cancer which relies on the detection of a truncated BSDL protein.

After having demonstrated that the insertional mutation of a cytosine into exon 11 of the *BSDL* gene leads to the expression of a truncated protein with a modified Ct-extremity characterized by the final PRAAHG sequence, the inventors have developed polyclonal antibodies against this BSDL variant. In a particular embodiment, they propose to use antibodies able to recognize specifically the PRAAHG Ct-extremity of a BSDL truncated protein to diagnose patient with PDAC.

Such a PRAAHG sequence in BSDL has already been characterized as a gene polymorphism of the milk counterpart of the pancreatic BSDL, but was not associated with any breast pathology [22]. Here the inventors demonstrated that the *BSDL* gene sequence modification has been detected within 15.6% of patients with PDAC that were examined.

Interestingly, anti-PRAAHG antibodies also recognize some tissue areas or cells of WT/WT genotyped patients with PDAC, suggesting that anti-PRAAHG antibodies are more appropriate to detect C-terminal sequence modification in BSDL than is the PCR/sequencing to detect the cytosine insertion in BSDL VNTR. Indeed, in the study presented in the present application, some 72% of tissues coming from patients with PDAC were reactive to anti-PRAAHG antibodies. Examination of the areas of tissue positive to anti-PRAAHG antibodies showed normal acinar morphology without apparent neoplasticity. Although this point requires confirmation, it could be that the apparent cytosine insertion in VNTR of the *BSDL* gene occurs early during carcinogenesis, in particular during Pancreatic Intraepithelial Neoplasia.

In the light of deleterious effects of truncated/mutated BSDL in the pancreas, the retention or recapture of which may cause diseases such as MODY-8 diabetes (due to inherited deletion in VNTR) [11, 12, 13, 14] or chronic pancreatitis (due to a recombined allele of the *BSDL* gene and the *BSDL* pseudogene) [15], the inventors examined the presence of the shorter isoform of BSDL in pancreatic juices from patients with PDAC. A response to the anti-PRAAHG antibodies was detected in pancreatic juices collected from WT/ApInsC genotyped patients and is associated with the truncated isoform of BSDL. This suggests, therefore, that the expression of the PRAAHG C-terminal sequence does not impact on BSDL protein expression and secretion, and may not affect cell behavior. However the exact role of this truncated isoform of BSDL in PDAC development requires further examination. The detection of the PRAAHG-reactive isoform of BSDL in pancreatic juice or pancreatic biopsies may be a new tool in PDAC diagnosis.

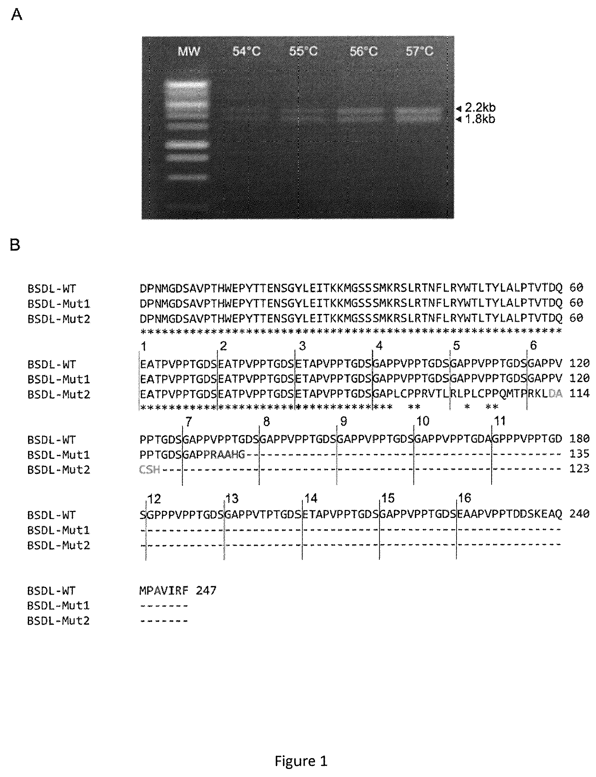

The sequences of the wild type and variant BSDL proteins Ct-extremities are provided on figures 1B.

For the generation of antibody binding specifically to the truncated BSDL protein, the nine carboxy-terminal amino acids of the protein were used; this amino acids sequence corresponds to GAPPRAAHG and referenced as SEQ ID NO 6. For the generation of antibody binding specifically to the wild-type BSDL protein, the twelve carboxy-terminal amino acids of the protein were used; this amino acids sequence corresponds to CKEAQMPAVIRF and referenced as SEQ ID NO 7.

Ad used herein, the BSDL wild-type protein corresponds to SEQ ID NO.5 (NP_001798.2) BSDL is secreted by the acinar cells of the pancreas and is present in the pancreatic juice. Thus, the detection of the truncated form of BSDL with PRAAHG extremity for diagnosis of pancreatic cancer can be performed in biological sample from a patient, including pancreatic juice, blood, serum, lymph, urine, stools... In a preferred embodiment, diagnosis will be performed on patient blood sample, preferably on blood serum.

An antibody of interest in the context of this invention is any antibody which specifically binds to the Ct-epitope of the truncated form of BSDL. This epitope can be defined as a linear epitope comprising the PRAAHG sequence, in particular the linear epitope can consist in GAPPRAAHG; this antibody must not bind to the wild-type form of BSDL.

As used herein, the term "antibody" comprises polyclonal antibodies, monoclonal antibodies or recombinant antibodies.

As used herein, a "monoclonal antibody" is intended to refer to a preparation of antibody molecules, antibodies which share a common heavy chain and common light chain amino acid sequence, in contrast with "polyclonal" antibody preparations which contain a mixture of antibodies of different amino acid sequence. Monoclonal antibodies can be generated by several known technologies like phage, bacteria, yeast or ribosomal display, as well as by classical methods exemplified by hybridoma-derived antibodies. Thus, the term "monoclonal" is used to refer to all antibodies derived from one nucleic acid clone.

The monoclonal antibodies of the present invention include recombinant antibodies. As used herein, the term "recombinant antibody" refers to antibodies which are produced, expressed, generated or isolated by recombinant means, such as antibodies which are expressed using a recombinant expression vector transfected into a host cell; antibodies isolated from a recombinant combinatorial antibody library; antibodies isolated from an animal (e.g. a mouse) which is transgenic due to human immunoglobulin genes (see, for example, Taylor, L.D., et al. (1992) Nucl. Acids Res. 20:6287-6295); or antibodies which are produced, expressed, generated or isolated in any other way in which particular immunoglobulin gene sequences (such as human immunoglobulin gene sequences) are assembled with other DNA sequences. Recombinant antibodies include, for example, chimeric, CDR graft and humanized antibodies.

The monoclonal antibodies of the present invention also include antigen-binding moiety. As used herein, the term "antigen-binding moiety" of an antibody (or simply "antibody moiety") refers to one or more fragments of an antibody of the invention, said fragment(s) still having the binding affinities as defined above. Fragments of a complete antibody have been shown to be able to carry out the antigen-binding function of an antibody. In accordance with the term "antigen-binding moiety" of an antibody, examples of binding fragments include (i) an Fab fragment, i.e. a monovalent fragment composed of the VL, VH, CL and CH1 domains; (ii) an F(ab')2 fragment, i.e. a bivalent fragment comprising two Fab fragments linked to one another in the hinge region via a disulfide bridge; (iii) an Fd fragment composed of the VH and CH1 domains; (iv) an Fv fragment composed of the FL and VH domains of a single arm of an antibody. Although the two domains of the Fv fragment, namely VL and VH, are encoded by separate genes, they may further be linked to one another using a synthetic linker, e.g. a poly-G4S amino acid sequence, and recombinant methods, making it possible to prepare them as a single protein chain in which the VL and VH regions combine in order to form monovalent molecules, known as single chain Fv (ScFv). The term "antigen- binding moiety" of an antibody is also intended to comprise such single chain antibodies. Other forms of single chain antibodies such as "diabodies" are likewise included here. Diabodies are bivalent, bispecific antibodies in which VH and VL domains are expressed on a single polypeptide chain, but using a linker which is too short for the two domains being able to combine on the same chain, thereby forcing said domains to pair with complementary domains of a different chain and to form two antigen-binding sites.

In the context on the invention, a preferred antibody is a monoclonal antibody allowing the specific recognition of the PRAAHG Ct-extremity of a BSDL truncated protein.

In another particularly embodiment, the antibody of the invention recognizing the Ct-extremity of a truncated BSDL protein is a non-naturally occurring antibody. As used herein, a "non-naturally occurring antibody" refers to an antibody that has been engineered by humans and includes humanized antibody, chimeric antibody, conjugated antibody or an antigen-binding moiety as defined above.

The antibody of the invention can be used in different methods to detect the expression of a truncated BSDL protein. These methods are well known form a skilled person in the art. They include immunodetection (western blot, immunofluorescence, immunochemistry...), imaging.

In a particular embodiment, the antibody contains a tag allowing the detection of the antibody. This tag can either be part of the recombinant antibody, as for example an His-tag containing at least 6 histidines, or be covalently linked to the antibody, as for example an enzyme. Such enzymes can be for example horseradish peroxidase (HRP) and alkaline phosphatase (AP), two enzymes commonly used in ELISA assays.

As shown in the experimental part, immunochemistry experiments on sections of pancreatic adenocarcinoma reveal that 70% of these PDAC patients express the truncated BSDL protein (See Example 4). This result confirms the relevance of this biomarker for the diagnosis of pancreatic cancer.

Furthermore, some of these patients expressing a truncated BSDL protein have previously been diagnosed as being homozygous wild type by genotyping of exon 11 of *BSDL* gene. Whereas it appears at first sight as a discrepancy, this result can be explained by considering that the (very) low quantity of mutated sequences present in the sample may be below the detection limit of the PCR method. However, interestingly, this result suggests that truncated BSDL may be expressed at an early stage in pancreatic cancer development and supports the fact that diagnosis using specific antibody is sensible, even while the number of mutation is still low. Accordingly, the detection of the expression of a BSDL with PRAAHG Ct-extremity can be proposed to identify patient with a pancreatic cancer at an early stage. This method may allow to detect patient while they are still asymptomatic. In this sense, the present invention provides opportunity for a new therapeutic approach for pancreatic cancer, since an early treatment is a first requirement for improving the therapeutic efficacy.

in another embodiment, the present invention an *in vitro* method for prognosis and/or diagnosis of a pancreatic cancer comprising the steps of (i) providing a patient sample, (ii) contacting said sample with an antibody specifically recognizes the PRAAHG Ct-extremity of a BSDL truncated protein and (iii) detecting the presence of said truncated BSDL protein in said sample. This method can further comprises the steps of administrating a treatment of pancreatic cancer when a truncated BSDL is present.

In a particular embodiment, the present invention concerns a kit for the detection of a cytosine insertion into a VNTR motif located in exon 11 of the *BSDL* gene in a patient biological sample.

Such kit can be presented in the format of a ready-to-use kit containing in particular the antibody able to specifically recognize the PRAAHG Ct-extremity of a BSDL truncated protein. Such is useful for the prognosis and/or diagnosis of a pancreatic cancer.

Said kit can be provided in the format of an ELISA assay for the detection of the presence of a truncated BSDL protein in in a biological sample of a patient such as blood, serum, pancreatic juice, lymph, urine, stools, preferably serum. An "ELISA assay" (enzyme-linked immunosorbent assay) is a plate-based assay technique designed for detecting and quantifying substances such as peptides, proteins, antibodies and hormones. In an ELISA, an antigen must be immobilized to a solid surface and then complexed with an antibody that is linked to an enzyme. Detection is accomplished by assessing the conjugated enzyme activity via incubation with a substrate to produce a measureable product.

The invention further concerns the use of a kit as previously defined for the prognosis and/or diagnosis of a pancreatic cancer.

The present invention also concerns the use of an antibody which specifically recognizes the PRAAHG Ct-extremity of a BSDL truncated protein for the *in vitro* prognosis and/or diagnosis of a pancreatic cancer.

In a further embodiment, the method of diagnosis of the invention also allows to establish a prognosis of pancreatic cancer evolution.

For example, this method can be used to follow the disease evolution after tumor resection or to identify potential relapse of the patients.

The experimental data provided thereafter illustrate the present invention but should not considered as limiting the scope of the invention.

### DESCRIPTION OF THE FIGURES

**Figure 1****. Genetic alterations within the genomic DNA sequence of BSDL.**
   A: Sequences encoding BSDL were amplified from the genomic DNA extracted from pancreatic tumor SOJ-6 cells using PCR and specific primers (Table 1). The PCR was performed in a personal Robocycler Gradient 96 (Stratagene). Following PCR, migration on agarose gel was performed to visualize amplified fragments.
   B: Amplicons were purified and their amino-acid sequences deduced after Sanger sequencing. The BSDL-WT amino-acid sequence corresponds to the 2.2 kb amplicon associated with the BSDL-Mut1 sequence, and the BSDL-Mut2 to that of the 1.8 kb band. Star symbol indicates the stop codon.

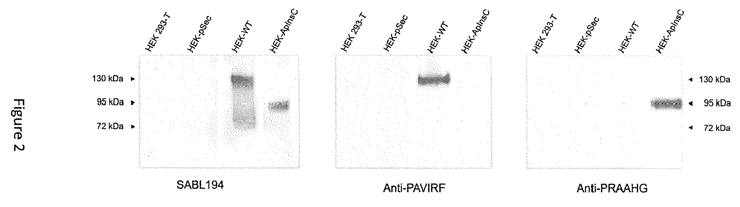

**Figure 2****. HEK293-T cell supernatants reactivity with SAB L194, anti-PAVIRF and anti-PRAAHG antibodies.**
   HEK293-T cells were transfected with transcripts coding for the wild-type BSDL inserted in the pSec-Tag vector (HEK-pSec-WT) or with transcripts encoding the ApInsC BSDL (HEK-pSec-ApInsC). Cells were cultured to confluence and supernatants were removed. Conditioned medium (30 µg protein per lane) was loaded for electrophoresis on SDS-PAGE gels and transferred onto a nitrocellulose membrane. After saturation, the membrane was incubated overnight with the primary antibody as indicated, then washed and incubated with the required secondary POD antibody before detection.

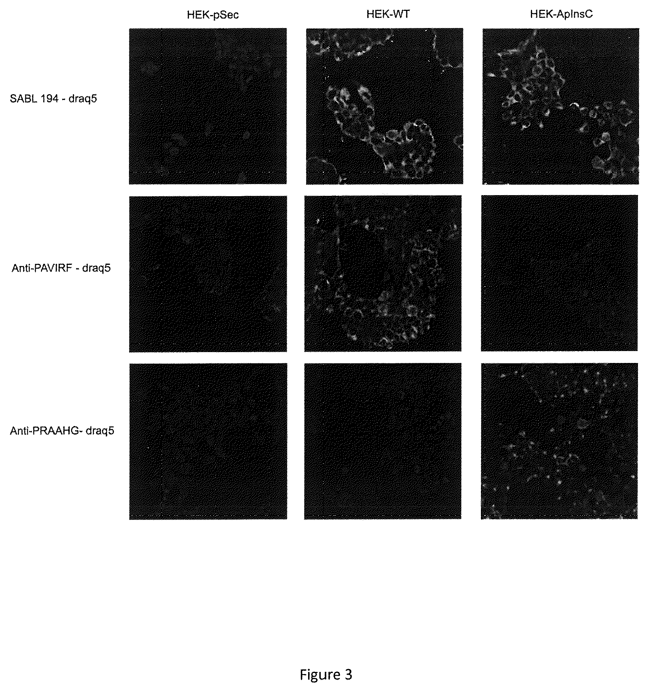

**Figure 3****. Transfected HEK293-T cells reactivity with SAB L194, anti-PAVIRF and anti-PRAAHG antibodies.**
   HEK293-T cells were transfected with empty pSecTag vector (HEK-pSec), with cDNA encoding the wild-type BSDL (HEK-WT) or with cDNA coding for the mutated BSDL (HEK-ApInsC), and seeded on 1.4 cm-diameter cover slips in 12-well plates. 48 hours later, cells were washed with PBS and then fixed and saturated, nuclei were blue-colored with Draq5 (1 µM, 10 min, 37°C) and saturated with 1% BSA. Cells were incubated with primary antibodies and bound antibodies were detected using a secondary antibody to rabbit IgG, coupled to Alexa488. Examination was performed using a SP5 Leica confocal microscope.

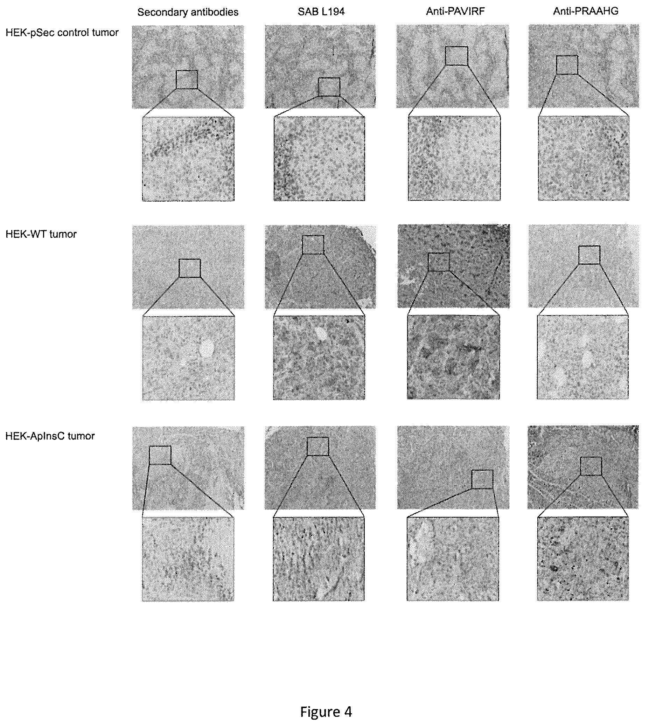

**Figure 4****. Immunohistochemistry on tumors xenatransplanted in nude mice.**
   HEK293-T cells transfected with empty pSecTag vector (HEK-pSec control tumor), with the cDNA encoding the wild-type BSDL (HEK-WT tumor), or with the cDNA coding for the mutated BSDL (HEK-ApInsC tumor) were xenotransplanted in nude mice. Tumors were excised at 500 mm³, then frozen and formalin-fixed. All immunohistochemistry steps were performed on the Leica BOND III automated system according to the manufacturers' instructions. Anti-PAVIRF and anti-PRAAHG antibodies were used at 0.2µg/ml and SAB L194 antibody at 1/1500 in Leica diluents.

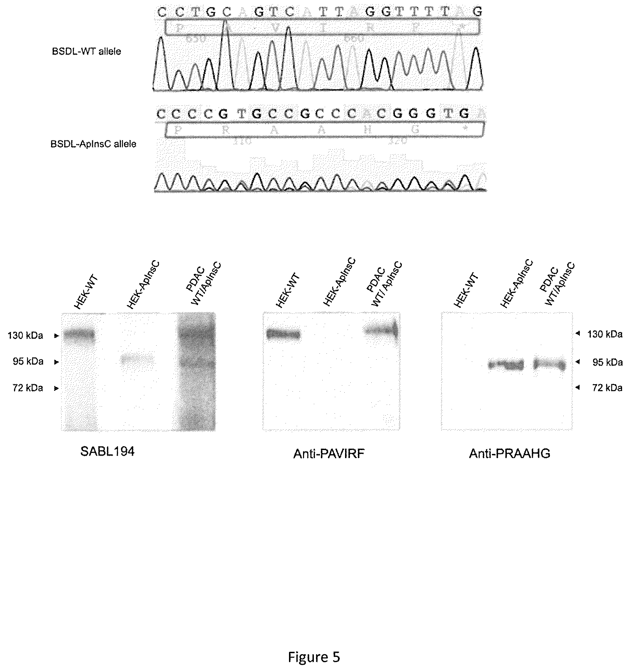

**Figure 5****. Immunoreactivity of human pancreatic tissue homogenates.**
   **A:** Analysis of electropherograms obtained after the sequencing of amplicons by the Sanger technique on samples from the above studied patients with wild-type allele or with the ApInsC allele. The electropherogram displays only the area where the apparent cytosine insertion occu rs.
   **B:** Pancreatic tumor tissue from a patient (WT/InsC genotyped) was homogenized before SDS-PAGE analysis (50 µg loaded on to the gel) and immunodetection with antibodies was as specified. The band migration was compared to that obtained with transfected HEK293-T cells (see Figure 2). The detection was performed with the required secondary POD antibody.

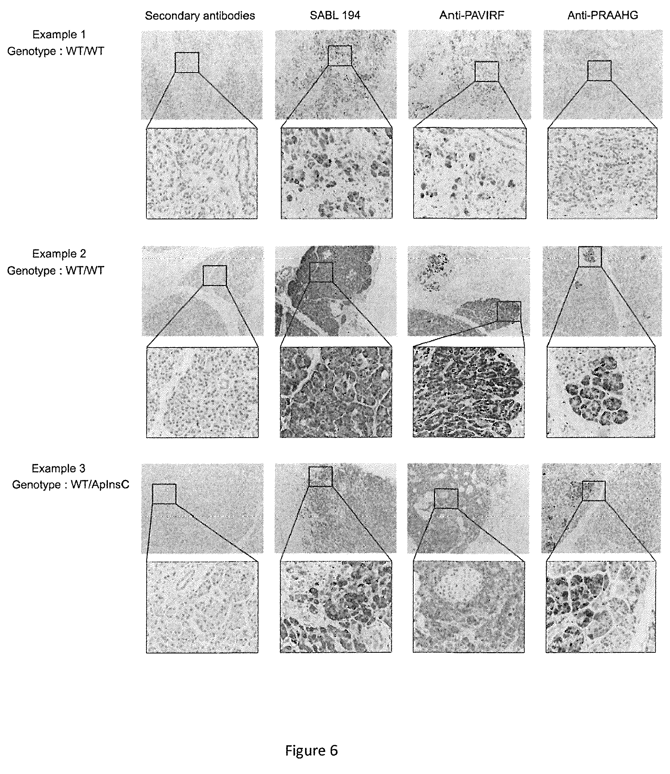

**Figure 6****. Immunohistochemistry on human pancreatic tumors.**
   Formalin-fixed, paraffin-embedded tissue sections of pancreatic tissue from genotyped WT/WT or WT/AplnsC patients were stained by anti-PAVIRF, anti-PRAAHG and SAB L194 antibodies. All immunohistochemistry steps were performed on the Leica BOND III automated system according to the manufacturers' instructions. Anti-PAVIRF and anti-PRAAHG antibodies were used at 0.2µg/ml and SAB L194 antibody at 1/1500 in Leica diluent.

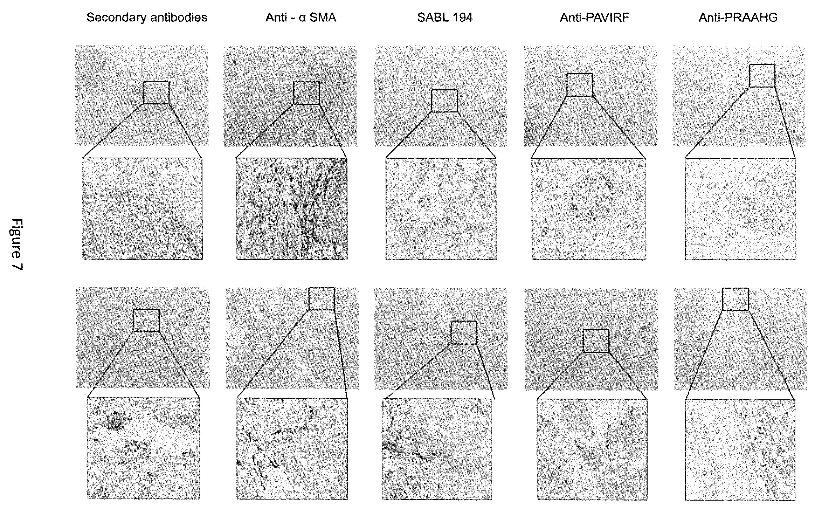

**Figure 7****. Immunohistochemistry on Control tissues.**
   Formalin-fixed, paraffin-embedded tissue sections of the stromal area of a PDAC tumor or of a neuro-endocrine tumor were stained by anti-PAVIRF, anti-PRAAHG, SAB L194 and anti α-SMA antibodies. All immunohistochemistry steps were performed on the Leica BOND III automated system according to the manufacturers' instructions. Anti-peptide (anti-PAVIRF and anti-PRAAHG) and anti α-SMA antibodies were used at 0.2µg/ml and 0.7 mg/ml, respectively. SAB L194 antibody was diluted at 1/1500 in Leica diluent.

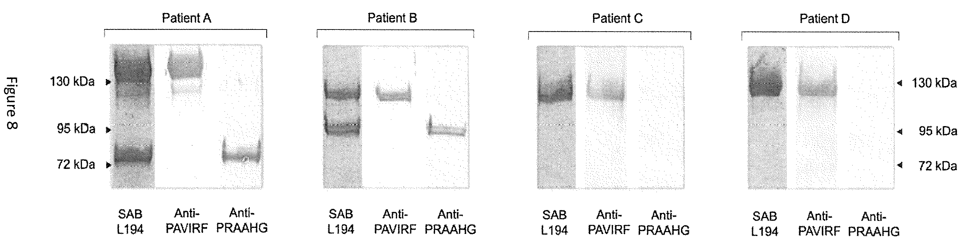

**Figure 8****. Immunoreactivity of human pancreatic juices.**
   Human pancreatic juice collected from patients A and B (WT/INsC genotyped) and from patients C and D (WT/WT genotyped) was analyzed on SDS-PAGE (approx. 50 µg protein loaded). Immunodetection was performed via incubation first with primary antibodies as specified then with the required secondary alkaline phosphatase antibodies.

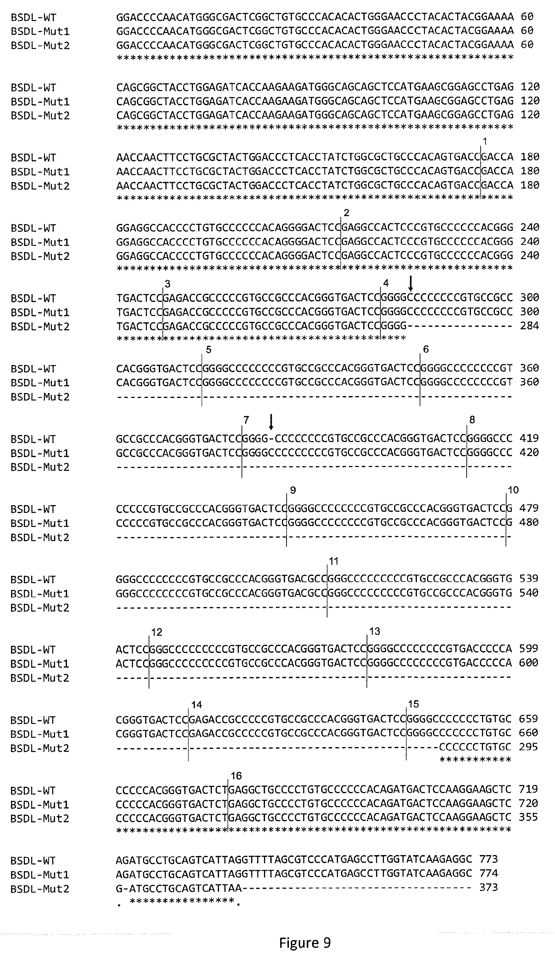

**Figure 9****. Nucleotide sequence of transcripts isolated from pancreatic tumor SOJ-6 cell line.**
   Amplifications of sequences encoding BSDL were performed by PCR from the genomic DNA extracted from pancreatic tumor SOJ-6 cells using specific primers (Table 1). The PCR was performed in a personal Mastercycler thermocycler. Following PCR, migration on agarose gel was performed to visualize the amplified fragment and check the size. Amplicons were purified and Sanger sequenced. The BSDL-WT sequence corresponds to the 2.2 kb amplicon associated with the BSDL-Mut1 sequence and the BSDL-Mut2 to that of the 1.8 kb band (see Figure 1). Number represents the repeated sequences in the VNTR of BSDL.

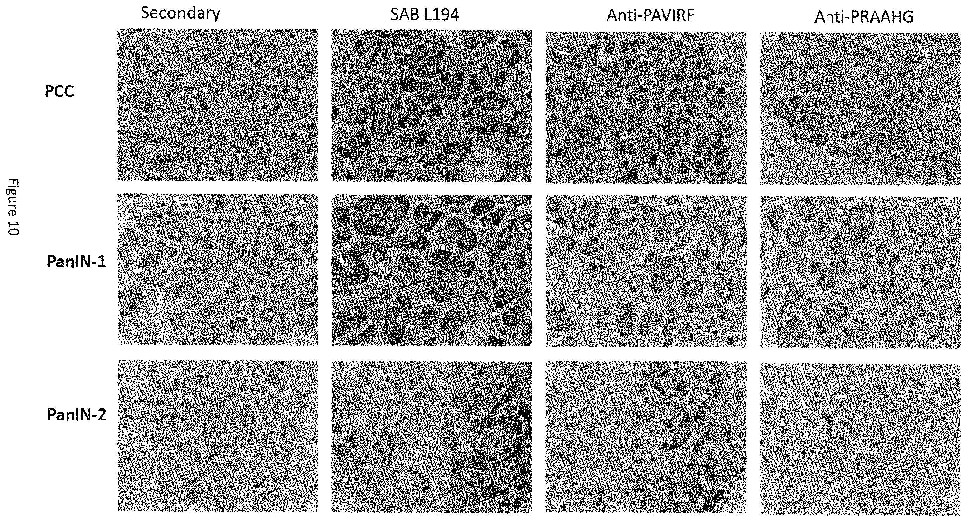

**Figure 10****. Immunohistochemistry on human pancreatic pretumoral tissues.**
   A tissue microarray (TMA; Euromedex, France) with tissues samples from patients with pancreatic cancer (PCC) and Pancreatic Intraephithelial Neoplasia (PanIN) were examined with anti-PAVIRF, anti-PRAAHG and SAB L194 antibodies. All immunohistochemistry steps were performed on the Leica BOND III automated system according to the manufacturers' instructions. Anti-PAVIRF and anti-PRAAHG antibodies were used at 0.2µg/ml and SAB L194 antibody at 1/1500 in Leica diluent.

### EXPERIMENTAL DATA

### MATERIAL AND METHODS

### Cell lines

SOJ-6 cells originating from human pancreatic adenocarcinoma were grown in RPMI 1640 medium supplemented with 10% fetal calf serum (FCS), penicillin (100 U/ml) and streptomycin (100 µg/ml). Human embryonic kidney (HEK) cells expressing the SV40 large T antigen (HEK293-T) (ATCC CRL-11268) were maintained in DMEM medium supplemented with 10% FCS, glutamine (2mM), penicillin (100 U/ml) and streptomycin (100 µg/ml),

### Antibodies

Anti-PRAAHG antibodies were generated in rabbit using the nine carboxy-terminal amino acids of BSDL-ApInsC (GAPPRAAHG). Anti-PAVIRF antibodies were generated in rabbit using the twelve carboxy-terminal amino acids of BSDL-WT (CKEAQMPAVIRF). Polyclonal rabbit antiserum SAB L194 detects all BSDL isoforms (Eurogentec, Angers, France). Alkaline phosphatase-labeled goat antibodies to rabbit IgG, peroxidase (POD)-labeled goat antibodies to rabbit IgG and AlexaFluor488-labeled donkey antibodies to rabbit IgG were purchased from LifeTechnologies (St Aubin, France). DRAQ5™, a far-red fluorescent DNA dye was from Biostatus Limited (Shepshed, UK). Antibodies to α-SMA (clone 1A4) came from DakoCytomation A/S (Glostrup, Denmark).

### Pancreatic cancer samples

Tumor tissues samples were obtained after pancreatic resection (duodeno-pancreatectomy) from 32 patients diagnosed with pancreatic adenocarcinoma (Gastroenterology and Digestive Surgery departments, Timone Hospital, Marseille) between February 2007 and February 2014 (Table 2). Patients were aged between 50 and 87 years (mean = 66.1 years, SD = 9.4) and the male/female ratio was 2/3. A definitive diagnosis of pancreatic adenocarcinoma was made after histochemical analysis of the resected tumor tissues. The average tumor size was 3.25 cm (SD = 1.2). WHO and TNM stages were determined by a senior pathologist with expertise in the field. One patient had a stage 1B localized tumor, 10 a stage 2A localized tumor, 16 stage 2B lymph node metastases, 3 were diagnosed at stage 4 (metastatic stage) and 2 were NC (data not communicated). Survival ranged from 3 to 72 months. According to the 2014 hospital records, 22 out of 32 patients with pancreatic cancer died and 10 were still alive at the time of the study. Information is lacking for the remaining patients. Whenever possible, pancreatic juice was collected during surgery and immediately frozen in liquid nitrogen.

### Non-malignant pancreatic disease samples

Patients displaying non-malignant pancreatic tissue with CCP or intraductal papillary mucinous tumor (IPMT) constituted the non-malignant pancreatic diseases (non-MPD) group. This cohort collected in the Gastroenterology department of Timone Hospital, Marseilles France, comprised 19 patients aged between 40 and 76 years (mean age = 65.1 years, SD = 11.3) (Table 3). The male/female ratio was 1.7/1. Four patients presented an IPMT, i.e. a cystic tumor of the pancreas with putative pre-neoplastic status, 14 had a CCP, and 1 presented a CCP complicated by a retention cyst. According to the hospital records, all these patients were still alive at the end of 2014.

### Non-pancreatic malignant disease samples

A cohort of 40 patients with non-pancreatic malignancies was examined. All 40 patients presented with glioblastoma. Tissue samples were collected in the Neurobiology department of Timone Hospital. Patients (male/female ratio, 1/3) were aged between 20 and 81 years (mean = 58.9 years, SD = 12.5, Table 4).

### Non-PDAC pancreatic cancers

The tissue samples were obtained after pancreatic resection (duodeno-pancreatectomy) in patients diagnosed with non-PDAC pancreatic cancers such as ampulloma, malignant endocrine carcinoma or mucinous cystadenoma (n = 6). These patients constituted the non-PDAC pancreatic cancer (non-PDAC) group (n = 6). Subjects were aged between 60 and 83 years (mean = 72.2 years, SD = 8.7) and the male/female ratio was 1/1 (Table 5). Control samples were collected in the Department of Gastroenterology and Digestive Surgery (Timone Hospital) between May 2009 and July 2013.

### Generalities on sampling

All tissue samples were from Caucasian subjects. Protocols for sample collection, sample anonymity, and genomic DNA analysis were approved by the local ethics committee. Written informed consent from all participants was obtained. Samples were stored in the CRO2 (Center for Research in Oncobiology and Oncopharmacology) biobank (agreement DC 2013-1857) or AP-HM (Assistance Publique - Hôpitaux de Marseille) BioBank (agreement AC-2013-1786). They were kept in a solution of RNA later ^{®} (Life Technologies) from immediately after surgery and then subjected to snap-freezing in liquid nitrogen.

### Extraction of total RNA from SOJ-6 cell line

The extraction of total RNA was performed using the RNeasy Plus Mini kit (Qiagen) according to the protocol recommended by the supplier. Total RNA was quantified by optical density at 260 nm on LVisPlate Omega (BMG Labtech). The quality and integrity of extracted RNA was verified by agarose gel migration. Total RNA samples were then stored at -80°C until use.

### Extraction of genomic DNA (gDNA) from whole blood, and tissue samples

The gDNA was extracted from whole blood or frozen tissue using the CIAamp mini kit (Qiagen). The extraction was carried out using 600 µl of whole blood or 25 mg of tissue as recommended by the supplier. The quantity and quality of extracted gDNA were determined by measuring the absorbance at 260 nm and 280 nm on LVisPlate Omega, gDNA samples were stored at -80°C until use.

### Amplification by RT-PCR

Reverse transcription was performed from 1 µg of total RNA in a reaction volume of 20 µl, containing 0.4 mM Oligo(dT)₁₅ primer, 2 mM dNTP mix, 5 mM MgCl2, 20 U RNase inhibitor and 10 U AMV reverse transcriptase (AMV Reverse Transcriptase kit, Promega). The mixture was incubated 10 min at room temperature (RT), followed by incubations: one hour at 42°C, 5 min at 99°C and finally 10 min on ice. Then, the PCR was performed in a personal Robocycler Gradient 96 (Stratagene) in a reaction volume of 50 µl and comprised 2 µl of cDNA, 5 µl of 10X enzyme buffer, 10 µl of 5X GCmelt buffer (Ozyme), 200 nM of each primer, 0.4 mM of dNTP mix and 5 U Platinum Taq High fidelity DNA polymerase (Life Technologies). The program was applied as follows: 1 cycle of 5 min at 94°C; 35 cycles of 1 min at 94°C, 1 min at 54-57°C, 4 min at 68°C; 1 cycle of 10 min at 68°C. Following PCR, migration on agarose gel was performed to visualize the amplified fragment and check the size.

### Amplifications by TD-PCR

The TD-PCR was performed in a personal Mastercycler thermocycler (Eppendorf) in a reaction volume of 50 µl and comprised 100 ng gDNA or 2 µl of cDNA, 5 µl of 10X enzyme buffer, 10 µl of 5X GCmelt buffer (Ozyme), 200 nM of each primer, 0.4 mM of dNTP mix and 5 U Platinum Taq High fidelity DNA polymerase (Life Technologies). The program was applied as follows: 1 cycle of 5 min at 94°C; 9 cycles of 30 sec at 94°C, 30 sec at 64°C (-1°C per cycle), 1 min at 68°C; 30 cycles of 30 sec at 94°C, 30 sec at 55°C, 1 min at 68°C; and 1 cycle of 12 min at 68°C. Following PCR, migration on agarose gel was performed to visualize the amplified fragment and check the size.

The primers used for amplification by RT-PCR and TD-PCR are detailed in Table 1.

**Table 1: Primers and probes used for amplification and detection of exon 11 BSDL gene**

| **Primers used for Sanger sequencing:** | | **Sequence number** |
|---|---|---|
| *Forward primers* | | |
| gDNA: | 5' TCTTCTCACTCTGCAGGGACC 3' | SEQ ID NO.1 |
| cDNA: | 5' GAACCAACTTCCTGCGCTAC 3' | SEQ ID NO.2 |

| *Reverse primer* | | |
|---|---|---|
| gDNA/cDNA: | 5' CAGGGGTATGAGGCTTTATTCA 3' | SEQ ID NO.3 |

### Amplicon purification and sequencing

The amplicons obtained were then purified using the DNA extraction kit (Millipore) after electrophoretic migration on agarose gel, according to the protocol recommended by the supplier. Sequencing of amplicons was carried out according to the Sanger method (Beckman Coulter Genomics, Meylan, France).

### Cell transfection

HEK293-T cells were transfected with pSecTag2B plasmid (Invitrogen) harboring BSDL-WT or BSDL-ApInsC using lipofectamine LTX (Life Technologies) as described by the manufacturer. Cells transfected with pSecTag2B without any insert were included in all experiments as a negative control. For stable BSDL expression, transfected HEK293-T cells were treated with 10 or 200 µg/ml Zeocin.

### Preparation of conditioned medium

HEK293-T cells were grown in DMEM medium supplemented with glutamine (2 mM), penicillin (100 U/ml), and streptomycin (100 µg/ml) to reach 60-70% confluence and then starved for 12 hours. Conditioned media were used for recombinant BSDL purification [23] and western blottings.

### Enzyme activity

Recombinant BSDL activity was recorded on a synthetic substrate as previously described [23].

### Immunofluorescence staining

Cells were seeded in appropriate medium on cover slips in 12-well plates (BD Falcon, Le Pont-de-Claix, France). Once adherent, cells were fixed (formaldehyde, 4% in PBS, at RT, 15 min) permeabilized with PBS-0.05% saponin (20 min at RT) and saturated (BSA, 4% in PBS, 30 min). The cells were then successively incubated with primary antibodies to PRAAHG or to PAVIRF peptides, or with SAB L194 antibody, for 120 min at RT and secondary AlexaFluor488-labeled antibodies to rabbit IgG for 60 min. The cell nuclei were labeled with 1 µM Draq5 for 30 min. Confocal microscopy acquisitions were performed using a Leica SP5 microscope coupled with a Leica scanning device (Leica Microsystems, Mannheim, Germany). Images were recorded with LAS AF Lite acquisition software and were analysed with the public-domain ImageJ software (NIH; http://rsb.info.nih.gov/nih-image/).

### Immunohistochemistry

Sections (5 µm) of formalin-fixed paraffin-embedded tissue were mounted on superfrost/plus slides from preheated water baths. All steps were performed on the Leica BOND III automated system (Leica Microsystems, Bannockburn, IL) according to the manufacturers' instructions. Anti-PAVIRF and anti-PRAAHG antibodies were used at 1/5000 in Leica diluents and SAB L194 antibody at 1/1500. The slides were dehydrated through 2 changes each of 70%, 95%, and 100% alcohol and 2 changes of xylene, before coverslipping.

### SDS-PAGE and western blottings

Proteins (30 µg of proteins from tissue homogenate or conditioned media or 5 µl of total pancreatic juice) in reducing SDS buffer were separated on 8% or 4-20% polyacrylamide gels (Thermo Scientific). After electrophoretic migration, proteins were transferred onto nitrocellulose membranes and processed for immunoblotting by using appropriate primary antibodies and alkaline-phophatase or POD-labeled secondary antibodies. After washes, membranes were developed with BCIP/NBT substrate or with a chemoluminescent substrate [24], visualized using a G:BOX-iChemi (SynGene) gel imaging device as described by the manufacturer, and analyzed with the NIH ImageJ program for band quantification.

### RESULTS

### Example 1: In vitro characterization of BSDL transcripts variants on SOJ-6 pancreatic cell line

To characterize *BSDL* transcripts in PDAC, specific primers of the full length *BSDL* cDNA were used to perform RT-PCR on genetic material extracted from the SOJ-6 pancreatic cell line, in which BSDL secretion is partly impaired [20]. Three transcripts were obtained and sequenced using Sanger's method. The first transcript, at approximately 2.2 kb, corresponded to the published sequence of the full length BSDL cDNA. An associated second sequence displayed an insertion of a cytosine residue at position 1885 in repeat 7 (BSDL-Mut1) leading to a premature stop codon and a different C-terminal sequence i.e. PRAAHG (Pro-Arg-Ala-Ala-His-Gly) (Figure 1). This differed from the PAVIRF (Pro-Ala-Val-Ile-Arg-Phe) found at the C-terminal end of the full-length wild-type BSDL (BSDL-WT), which represents the normal C-terminal amino-acid sequence of human BSDL The third transcript of 1.8 kb presented a cytosine residue deletion at nt1785. This mutation gives rise to a deletion of 330bp and possibly corresponds to the sequence deletion observed by Pasqualini et al. [18], with a premature stop codon and different C-terminal sequences (Figure 1) i.e. DACSH (Asp-Ala-Cys-Ser-His) for BSDL-Mut2. The full sequences of the three transcripts are given in Figure 9.

### Example 2: In vitro confirmation of the existence of three BSDL transcripts variants on pancreatic tissues from patients.

To confirm data obtained using human pancreatic tumor cell lines may not reflect physio-pathological situations, pancreatic tissue samples from a cohort of 97 patients were collected. Of these, 32 were patients with resected PDAC, 6 were patients with non-PDAC pancreatic cancers (ampulloma, malignant endocrine carcinoma, mucinous cystadenoma), 19 were patients with non-malignant pancreatic diseases (intraductal papillary mucinous tumor of the pancreas [IPMT] or chronic calcifying pancreatitis [CCP]), and 40 were patients with a non-pancreatic malignancy (glioblastoma) (see Tables 2-5 below for clinical data). Touch-down (TD)-PCR then allowed the amplification of genetic material extracted from tissues, using two pairs of primers, targeting the full exon 11 from 5'-end to 3'-end and the overlapping region between intron 10/exon 11 and 3'-end of exon 11 (Table 1).
Sequencing of the resulting transcripts indicated that 40/40 patients with glioblastoma and 6/6 patients with non-PDAC pancreatic cancers showed a wild-type amplification transcript of BSDL, and 1/19 patient with CCP *(i.e.* 5.3%) showed an insertion of a cytosine residue in repeat number 8. Five out of 32 patients (*i.e.* 15.6%) with PDAC showed an insertion of a cytosine residue in repeat 4, repeat 5, or repeat 9. Whatever the position of the cytosine residue insertion, this generated a premature stop codon and the same translated C-terminal sequence (*i.e* PRAAHG). This sequence correlated with that of BSDL-Mut1 characterized in SOJ-6 cells, and will now be referred to as *'BSDL* gene with an apparent cytosine insertion' or BSDL-ApInsC.

**Table 2. Clinical data of patients with pancreatic ductal adenocarcinoma.**

| Patients | Disease | Age¹ | Gender² | Tumor (diameter³) | TNM⁴ | | | Grade⁵ |
|---|---|---|---|---|---|---|---|---|
| | | | | | T | N | M | |
| PDAC 1 | PDAC | 59 | 0 | 4.5 | 2 | 1 | 0 | 2B |
| PDAC 2 | PDAC | 66 | 0 | 3.5 | 3 | 0 | 0 | 2A |
| PDAC 3 | PDAC | 54 | 0 | 3 | 3 | 1 | 0 | 4 |
| PDAC 4 | PDAC | 73 | 1 | 1.8 | 3 | 1 | 0 | 2B |
| PDAC 5 | PDAC | 62 | 0 | 2 | 3 | 1 | 0 | 2B |
| PDAC 6 | PDAC | 57 | 1 | 4.3 | 4 | 1 | 1 | 4 |
| PDAC 7 | PDAC | 70 | 0 | 2.3 | 3 | 1 | 0 | 2B |
| PDAC 8 | PDAC | 63 | 1 | 2.5 | 3 | 1 | 0 | 2B |
| PDAC 9 | PDAC | 66 | 0 | 6 | 3 | 0 | 0 | 2A |
| PDAC 10 | PDAC | 58 | 1 | 5.2 | 2 | 1 | 0 | 2B |
| PDAC 11 | PDAC | 67 | 1 | 1.5 | 3 | 0 | 0 | 2A |
| PDAC 12 | PDAC | 64 | 0 | 4.7 | 3 | 1 | 1 | 4 |
| PDAC 13 | PDAC | 57 | 1 | 6 | 3 | 1 | 0 | 2B |
| PDAC 14 | PDAC | 63 | 1 | 4.5 | 3 | 0 | 0 | 2B |
| PDAC 15 | PDAC | 66 | 1 | 2.5 | 3 | 0 | 0 | 2A |
| PDAC 16 | PDAC | 66 | 1 | 2.5 | 3 | 0 | 0 | 2A |
| PDAC 17 | PDAC | 79 | 1 | 2 | 3 | 0 | 0 | 2A |
| PDAC 18 | PDAC | 57 | 0 | 3 | 3 | 0 | 0 | 2A |
| PDAC 19 | PDAC | 62 | 0 | 3 | 3 | 0 | 0 | 2A |
| PDAC 20 | PDAC | 53 | 1 | 2 | 1 | 0 | 0 | 2A |
| PDAC 21 | PDAC | 61 | 1 | 2.3 | 3 | 1 | 0 | 2B |
| PDAC 22 | PDAC | 72 | 0 | 3 | 3 | 1 | 0 | 2B |
| PDAC 23 | PDAC | 69 | 1 | 3.5 | 3 | 0 | 0 | 2A |
| PDAC 24 | PDAC | 57 | 1 | 3.7 | 3 | 1 | 0 | 2B |
| PDAC 25 | PDAC | 73 | 1 | 2 | 2 | 0 | 0 | 1B |
| PDAC 26 | PDAC | 50 | 1 | 3 | 2 | 1 | 0 | 2B |
| PDAC 27 | PDAC | 87 | 1 | 3 | 3 | 1 | 0 | 2B |
| PDAC 28 | PDAC | 85 | 0 | 3.3 | 3 | 1 | 0 | 2B |
| PDAC 29 | PDAC | 79 | 1 | 3.5 | 3 | 1 | 0 | 2B |
| PDAC 30 | PDAC | 73 | 1 | 4.1 | 3 | 1 | 0 | 2B |
| PDAC 31 | PDAC | 81 | 0 | 2.4 | 3 | 1 | 0 | 2B |
| PDAC 32 | PDAC | 76 | 0 | NC | 2 | 0 | 0 | 1B |

| | | | | | | | | |
|---|---|---|---|---|---|---|---|---|
| PDAC: Pancreatic Ductal AdenoCarcinoma.. NC: data not communicated. ¹: in years. ²: Male 0, Female 1. ³: in centimeters. ⁴: TNM stage: T: Tumor, N: Node, M: Metastasis. ⁵: Tumor grade (WHO). | | | | | | | | |

**Table 3. Clinical data of the cohort of patients with non-malignant pancreatic diseases (non-MPD)**

| Patients | Diseases | Age¹ | Gender² |
|---|---|---|---|
| non-MPD 1 | IPMT | 69 | 0 |
| non-MPD 2 | IPMT | 56 | 0 |
| non-MPD 3 | CCP | 61 | 0 |
| non-MPD 4 | CCP | 72 | 0 |
| non-MPD 5 | CCP | 71 | 1 |
| non-MPD 6 | RT | 76 | 1 |
| non-MPD 7 | CCP | 40 | 1 |
| non-MPD 8 | CCP | 73 | 0 |
| non-MPD 9 | CCP | 53 | 1 |
| non-MPD 10 | IPMT | 74 | 1 |
| non-MPD 11 | IPMT | 71 | 0 |
| non-MPD 12 | CCP | 48 | 0 |
| non-MPD 13 | CCP | 53 | 1 |
| non-MPD 14 | CCP | 54 | 0 |
| non-MPD 15 | CCP | 68 | 0 |
| non-MPD 16 | CCP | 45 | 0 |
| non-MPD 17 | CCP | 69 | 1 |
| non-MPD 18 | CCP | 43 | 0 |
| non-MPD 19 | CCP | 62 | 0 |

| | | | |
|---|---|---|---|
| IPMT : intra-papillary mucinous tumor of the pancreas. CCP: chronic calcifying pancreatitis RT: Retention cyst. ¹: years. ²: Male: 0, Female: 1. | | | |

**Table 4. Clinical data of the cohort of patients with non-pancreatic malignancies**

| Patients | Diseases | Age¹ | Gender² | Grade³ |
|---|---|---|---|---|
| OC 1 | GB | 78 | 0 | 4 |
| OC 2 | GB | 20 | 0 | 4 |
| OC 3 | GB | 59 | 0 | 4 |
| OC 4 | GB | 52 | 0 | 4 |
| OC 5 | GB | 51 | 0 | 4 |
| OC 6 | GB | 63 | 0 | 4 |
| OC 7 | GB | 76 | 0 | 4 |
| OC 8 | GB | 48 | 0 | 4 |
| OC 9 | GB | 72 | 0 | 4 |
| OC 10 | GB | 56 | 0 | 4 |
| OC 11 | GB | 54 | 0 | 4 |
| OC 12 | GB | 39 | 0 | 4 |
| OC 13 | GB | 37 | 1 | 4 |
| OC 14 | GB | 75 | 0 | 4 |
| OC 15 | GB | 68 | 0 | 4 |
| OC 16 | GB | 46 | 0 | 4 |
| OC 17 | GB | 77 | 1 | 4 |
| OC 18 | GB | 49 | 0 | 4 |
| OC 19 | GB | 48 | 1 | 4 |
| OC 20 | GB | 59 | 1 | 4 |
| OC 21 | GB | 68 | 0 | 4 |
| OC 22 | GB | 53 | 0 | 4 |
| OC 23 | GB | 53 | 0 | 4 |
| OC 24 | GB | 59 | 1 | 4 |
| OC 25 | GB | 69 | 0 | 4 |
| OC 26 | GB | 73 | 0 | 4 |
| OC 27 | GB | 63 | 1 | 4 |
| OC 28 | GB | 70 | 1 | 4 |
| OC 29 | GB | 51 | 1 | 4 |
| OC 30 | GB | 69 | 0 | 4 |
| OC 31 | GB | 67 | 0 | 4 |
| OC 32 | GB | 54 | 1 | 4 |
| OC 33 | GB | 64 | 0 | 4 |
| OC 34 | GB | 75 | 0 | 4 |
| OC 35 | GB | 45 | 1 | 4 |
| OC 36 | GB | 59 | 0 | 4 |
| OC 37 | GB | 68 | 0 | 4 |
| OC 38 | GB | 61 | 0 | 4 |
| OC 39 | GB | 55 | 0 | 4 |
| OC 40 | GB | 51 | 0 | 4 |

| | | | | |
|---|---|---|---|---|
| GB: Glioblastoma. 1: in years. 2 : Male 0, Female 1. 3: Tumor grade (WHO). | | | | |

**Table 5. Clinical data of the cohort of patients with non-PDAC pancreatic cancers**

| Patients | Diseases | Age¹ | Gender² | Tumor (diameter³) | TNM⁴ | | | Grade⁵ |
|---|---|---|---|---|---|---|---|---|
| | | | | | T | N | M | |
| Non-PDAC 1 | MC | 69 | 0 | NC | | NC | | NC |
| Non-PDAC 2 | MA | 73 | 1 | 2.4 | 4 | 1 | 0 | NC |
| Non-PDAC 3 | EC | 81 | 1 | 3 | 3 | 1 | 0 | NC |
| Non-PDAC 4 | EC | 67 | 0 | 1.3 | 3 | 1 | 0 | 2B |
| Non-PDAC 5 | MA | 60 | 1 | 2.5 | 4 | 1 | 0 | NC |
| Non-PDAC 6 | EC | 83 | 0 | 6.5 | | NC | | 2B |

| | | | | | | | | |
|---|---|---|---|---|---|---|---|---|
| MC: Mucinous Cystadenoma. MA: Malignant Ampulloma. EC: Endocrine Carcinoma. NC: data not communicated. 1 : in years. 2 : Male 0, Female 1. 3 : in centimeters. 4 : TNM stage: T: Tumor, N: Node, M: Metastasis. 5 : Tumor grade (WHO). | | | | | | | | |

### Example 3: Preparation and validation of polyclonal antibodies specific of the WT and truncated BSDL proteins.

Because of the differences between the C-terminal sequences of BSDL-ApInsC and of BSDL-WT, polyclonal antibodies directed against the whole BSDL, against the C-terminal sequence of the BSDL-WT, and against that deduced from the nucleotide sequence of the Mut1 variant (BSDL-ApInsC) were generated. These polyclonal antibodies are referred to as SAB L194, anti-PAVIRF and anti-PRAAHG, respectively, and were reactive both with SOJ-6 cells and with tumors obtained after xenotransplantation of SOJ-6 cells in nude mice (data not shown). For recombinant protein expression, we cloned and inserted the cDNA of BSDL-WT and BSDL-ApInsC within the pSecTag2-vector for transfection into HEK293-T cells. These transfected cells expressed recombinant full-length BSDL (BSDL-WT) and mutated BSDL (BSDL-ApInsC) in cell supernatant, both of which were recognized by SAB L194 polyclonal antibody to human BSDL (Figure 2). Recombinant BSDL-WT and BSDL-ApInsC displayed identical specific activity on a synthetic substrate and did not affect HEK293-T cell growth. However, antibodies to PAVIRF recognized only BSDL-WT (Mr approx.. 120-130 kDa) and antibodies to PRAAHG bound only to BSDL-ApInsC (Mr approx 80-90 kDa). Immunohistochemistry also showed that HEK293-T cells expressing BSDL-WT (HEK-WT) were reactive to anti-PAVIRF antibodies whereas HEK293-T cells expressing BSDL-ApInsC (HEK-ApInsC) were recognized by anti-PRAAHG antibodies (Figure 3). Subsequently, we xenotranspianted HEK293-T cells into nude mice and resected the tumor when palpable for use in immunohistochemistry. Control tumors (i.e. tumors obtained after transplantation of HEK293-T cells transfected with the empty pSecTag2-vector) were not reactive to SAB L194, anti-PAVIRF or anti-PRAAHG antibodies. However, tumors recovered from mice xenotransplanted with HEK-WT and HEK-ApInsC cells were both reactive to SAB L194 and also respectively to anti-PAVIRF or anti-PRAAHG antibodies (Figure 4).
Taken together these data confirm the specificity of antibodies and their usefulness in both western-blotting and immunohistochemistry experiments.

### Example 4: Analysis of expression of BSDL in patients by immunochemistry

Investigations were pursued using human pancreatic tumor tissue sections. Tumor tissue from a patient presenting with both the wild-type allele (BSDL-WT) and an allele with an insertion of a cytosine residue in repeat Nb 9 (BSDL-ApInsC), therefore a WT/ApInsC genotype, was reactive to SAB L194, anti-PAVIRF, and anti-PRAAHG antibodies. Tissue from another patient homozygous for the wild-type allele (WT/WT genotype) was reactive to SAB L194 and to anti-PAVIRF antibodies but not to anti-PRAAHG antibodies (Figure 5). We therefore examined tissue sections from 18 patients with PDAC for reactivity to SAB L194, anti-PAVIRF and anti-PRAAHG antibodies. Fifty percent of these patients displayed a mutation on exon 2 of *KRAS.* Tissue sections from 5 out of 18 patients (27.8%) were WT/WT genotyped (see Figure 6 for an example) and also showed reactivity to SAB L194 and to anti-PAVIRF antibodies but not to anti-PRAAHG antibodies. Tissue sections from 5/18 (27.8%) patients were genotyped WT/ApInsC and were also reactive to SAB L194, anti-PAVIRF and anti-PRAAHG antibodies (see Figure 6 for an example). Interestingly, tissue sections from the remaining 8/18 (44.4%) patients were WT/WT genotyped and reactive to SAB L194 and to anti-PAVIRF antibodies but also displayed sporadic areas (some cells or small areas) that were reactive to anti-PRAAHG antibodies. These cells or areas, positive to anti-PRAAHG antibodies, presented a morphological acinar aspect that was not neoplastic, suggesting that truncated BSDL may be expressed early during pancreatic carcinogenesis.
Taken as a whole, 13/18 patients with PDAC (*i.e*. 72.2%) were positive to SAB L194, anti-PAVIRF and to anti-PRAAHG antibodies meaning that the truncated isoform of BSDL (BSDL-ApInsC), along with the normal form of the enzyme, is expressed in these tissues or at least by some cells within the tumor mass. Four patients genotyped WT/WT were not reactive to SAB L194, anti-PAVIRF or to anti-PRAAHG antibodies and tissue examination showed that these samples were mainly stromal (reactive to anti-α-SMA antibodies) with an absence of acinar tissue. Tissues from two patients with pancreatic neuro-endocrine tumors were not reactive to SAB L194, anti-PAVIRF, or to anti-PRAAHG antibodies (Figure 7).

### Example 5: Analysis of the expression of WT and truncated BSDL protein by western blot

Deletion of the C-terminal end of BSDL significantly decreased the expression of the protein [21]. SOJ-6 cells, from which BSDL-AplnsC (BSDL-Mut1) was isolated, also poorly secreted the enzyme. Although this poor secretion has been attributed to a defect in vesicular transport of secretory proteins [20], it is tempting to speculate that the BSDL-ApInsC isoform may not be secreted by acinar cells. To check this specific point, pancreatic juice samples were collected during surgery and analyzed by western blot using antibodies generated in our laboratory (Figure 8). Four patients with PDAC were examined; two with the WT/ApInsC genotype (patients A and B) and two with the WT/WT genotype (patients C and D). Samples from patients A and B (WT/ApInsC) displayed two bands around 120-130 and 75-95 kDa, both reactive with SAB L194 antibodies. Interestingly the lower band was reactive with anti-PRAAHG antibodies whereas the upper band was only recognized by anti-PAVIRF antibodies. In WT/WT genotyped patients C and D, only the higher band could be detected, associated with reactivity to SAB L194 and anti-PAVIRF antibodies. Important control individuals presenting with an allelic polymorphism with a various number of repeated sequences [9] were reactive only to SAB L194 and anti-PAVRIF antibodies (data not shown).

These data demonstrate that antibodies to PRAAHG peptide sequence were only reactive with BSDL-ApinsC, and that this short isoform of BSDL is secreted in pancreatic juice and appears to be specifically expressed in PDAC.

### Example 6: Analysis of the expression of truncated BSDL in preneoplastic pancreatic pathologies.

To investigate the expression of truncated BSDL in preneoplastic pancreatic pathologies, a tissue microarray (TMA) from Euromedex (France) was analyzed by immunohistochemistry (Figure 10). This commercial TMA contained 10 tissues samples from patients with Pancreatic Cancer (PCC) and 17 patients with Pancreatic Intraepithelial Neoplasia (PanIN), including 10 patients with PanIN-1 and 7 patients with PanIN-2. Among patients with PCC 30% were reactive to antibodies to PRAAHG while 7 patients over 10 patients afflicted with a PanIN-1 (70%) and 7/7 (100%) patients with a PanIN-2 were positive to antibodies to PRAAHG (Table 6). All these tissues were reactive to antibodies to PAVIRF and to SAB L194.

These data show that the expression of a truncated BSDL can be used for the diagnosis of pancreatic cancer at an early stage, in particular at stage of Pancreatic Intraephithelial Neoplasia.

**Table 6.**

| | | IHC positive labelling | | |
|---|---|---|---|---|
| Pathology | Number of patients | SAB L194 | Anti-PAVIRF | Anti-PRAAHG |
| PCC | 10 | 100% | 100% | 30% |
| PanIN-1 | 10 | 100% | 100% | 70% |
| PanIN-2 | 7 | 100% | 100% | 100% |

| | | | | |
|---|---|---|---|---|
| PCC: Prostate cancer; PanIN-1: Pancreatic Intraephithelial Neoplasia-1; PanIN-2: Pancreatic Intraephithelial Neoplasia-2; IHC: Immunohistochemistry | | | | |

### REFERENCES

1. Lombardo D. Bile salt-dependent lipase: its pathophysiological implications. Biochimica Biophysica Acta. 2001; 1533: 1-28. 1
2. Bläckberg L, Lombardo D, Hernell O, Guy O, Olivecrona T. Bile salt-stimulated lipase in human milk and carboxyl ester hydrolase in pancreatic juice: are they identical enzymes? FEBS Letter. 1981; 136: 284-288. 2
3. Lidberg U, Nilsson J, Strömberg K, Stenman G, Sahlin P, Enerbäck S, Bjursell G. Genomic organization, sequence analysis, and chromosomal localization of the human carboxyl ester lipase (CEL) gene and a CEL-like (CELL) gene. Genomics. 1992; 13: 630-640. 3
4. Taylor AK, Zambaux JL, Klisak I, Mohandas T, Sparkes RS, Schotz MC, Lusis AJ. Carboxyl ester lipase: a highly polymorphic locus on human chromosome 9qter. Genomics. 1991; 10: 425-431. 4
5. Nilsson J, Bläckberg L, Carlsson P, Enerbäck S, Hernell O, Bjursell G. cDNA cloning of human-milk bile-salt-stimulated lipase and evidence for its identity to pancreatic carboxylic ester hydrolase. European Journal of Biochemistry. 1990; 192: 543-550. 5
6. Baba T, Downs D, Jackson KW, Tang J, Wang CS. Structure of human milk bile salt activated lipase. Biochemistry. 1991; 30: 500-510. 6
7. Wang CS, Dashti A, Jackson KW, Yeh JC, Cummings RD, Tang J. Isolation and characterization of human milk bile salt-activated lipase C-tail fragment. Biochemistry. 1995; 34: 10639-10644. 7
8. DiPersio LP, Carter CP, Hui DY. Exon 11 of the rat cholesterol esterase gene encodes domains important for intracellular processing and bile salt-modulated activity of the protein. Biochemistry. 1994; 33: 3442-3448. 8
9. Strömqvist M, Hernell O, Hansson L, Lindgren K, Skytt A, Lundberg L, Lidmer AS, Bläckberg L. Naturally occurring variants of human milk bile salt-stimulated lipase. Archive of Biochemistry and Biophysics. 1997; 347: 30-36. 9
10. Swan JS, Hoffman MM, Lord MK, Poechmann JL. Two forms of human milk bile-salt-stimulated lipase. Biochemical Journal. 1992; 283: 119-122. 10
11. Torsvik J, Johansson S, Johansen A, Ek J, Minton J, Raeder H, Ellard S, Hattersley A, Pedersen O, Hansen T, Molven A, Njølstad PR. Mutations in the VNTR of the carboxyl-ester lipase gene (CEL) are a rare cause of monogenic diabetes. Human Genetics. 2010; 127: 55-64. 20
12. Raeder H, Johansson S, Holm PI, Haldorsen IS, Mas E, Sbarra V, Nermoen I, Eide SA, Grevle L, Bjørkhaug L, Sagen JV, Aksnes L, Søvik O, et al. Mutations in the CEL VNTR cause a syndrome of diabetes and pancreatic exocrine dysfunction. Nature Genetics. 2006; 38: 54-62. 21
13. Torsvik J, Johansson BB, Dalva M, Marie M, Fjeld K, Johansson S, Bjørkøy G, Saraste J, Njølstad PR, Molven A Endocytosis of secreted carboxyl ester lipase in a syndrome of diabetes and pancreatic exocrine dysfunction. Journal of Biological Chemistry. 2014; 289: 29097-29111. 23
14. Johansson BB, Torsvik J, Bjørkhaug L, Vesterhus M, Ragvin A, Tjora E, Fjeld K, Hoem D, Johansson S, Raeder H, Lindquist S, Hernell O, Cnop M, et al. Diabetes and pancreatic exocrine dysfunction due to mutations in the carboxyl ester lipase gene-maturity onset diabetes of the young (CEL-MODY): a protein misfolding disease. Journal of Biological Chemistry. 2011; 286: 34593-34605. 24
15. Fjeld K, Weiss FU, Lasher D, Rosendahl J, Chen JM, Johansson BB, Kirsten H, Ruffert C, Masson E, Steine SJ, Bugert P, Cnop M, et al. A recombined allele of the lipase gene CEL and its pseudogene CELP confers susceptibility to chronic pancreatitis. Nature Genetics. 2015; 47: 518-522. 25
16. Meyer JG. Lipolytic enzymes of the human pancreas. II. Purification and properties of cholesterol ester hydrolase. Biochimica Biophysica Acta. 1989; 1002 :89-92. 26
17. Duan RD, Borgström B. Is there a specific lysophospholipase in human pancreatic juice? Biochimica Biophysica Acta. 1993; 1167: 326-330. 27
18. Pasqualini E, Caillol N, Panicot L, Valette A, Lombardo D. Expression of a 70-kDa immunoreactive form of bile salt-dependent lipase by human pancreatic tumoral mia PaCa-2 cells. Archive of Biochemistry and Biophysics. 2000; 375: 90-100. 28
19. Kirby A, Gnirke A, Jaffe DB, Barešová V, Pochet N, Blumenstiel B, Ye C, Aird D, Stevens C, Robinson JT, Cabili MN, Gat-Viks I, Kelliher E, et al. Mutations causing medullary cystic kidney disease type (MCKD1) lie in a large VNTR in MUC1 missed by massively parallel sequencing. Nature Genetics. 2013; 45: 299-303. 29
20. Caillol N, Pasqualini E, Lloubes R, Lombardo D. Impairment of bile salt-dependent lipase secretion in human pancreatic tumoral SOJ-6 cells. Journal of Cell Biochemistry. 2000; 79: 628-647. 30
21. Hansson L, Bläckberg L, Edlund M, Lundberg L, Strömqvist M, Hernell. Recombinant human milk bile salt-stimulated lipase. Catalytic activity is retained in the absence of glycosylation and the unique proline-rich repeats. Journal of Biological Chemistry. 1993; 268: 26692-26698. 31
22. Lindquist S, Bläckberg L, Hernell O. Human bile salt-stimulated lipase has a high frequency of size variation due to a hypervariable region in exon 11. European Journal of Biochemistry. 2002; 269: 759-767. 75
23. Lombardo D, Guy O, Figarella C. Purification and characterization of a carboxyl ester hydrolase from human pancreatic juice. Biochimica Biophysica Acta. 1978; 527: 142-149. 76
24. Ristorcelli E, Beraud E, Verrando P, Villard C, Lafitte D, Sbarra V, Lombardo D, Verine A. Human tumoral nanoparticles induce apoptosis of pancreatic cancer cells. FASEB Journal. 2008; 22: 3358-3369. 77

## Claims

1. An *in vitro* method for the prognosis and/or diagnosis of a pancreatic cancer comprising the detection of a *BSDL* gene with an apparent cytosine insertion in a patient sample.

2. The method of claim 1, wherein the detection of said *BSDL* gene with an apparent cytosine consists in identifying a cytosine insertion into a VNTR located in exon 11 of the *BSDL* gene.

3. The method of claim 1, wherein the detection of said *BSDL* gene with an apparent cytosine consists in identifying the presence of a truncated BSDL protein.

4. The method of claim 2, wherein the detection of the cytosine insertion is performed on circulating tumoral DNA.

5. The method of claim 3, wherein the detection of said truncated BSDL protein is performed by using an antibody which specifically recognizes the PRAAHG Ct-extremity of a BSDL truncated protein.

6. An *in vitro* method for prognosis and/or diagnosis of a pancreatic cancer comprising the steps of (i) providing a patient sample, (ii) amplifying the VNTR regions of the exon 11 of the *BSDL* gene or the corresponding sequences in mRNA and (iii) detecting the presence of a cytosine insertion in the said gene.

7. The method of claim 6, further comprising the steps of administrating a treatment of pancreatic cancer when a cytosine insertion is present in at least one of said VNTR regions.

8. The method of any one of claims 1, 2, 4, 6 or 7, wherein said cytosine insertion occurs in VNTR 4, 5, 7 or 9.

9. An *in vitro* method for prognosis and/or diagnosis of a pancreatic cancer comprising the steps of (i) providing a patient sample, (ii) contacting said sample with an antibody which specifically recognizes the PRAAHG Ct-extremity of a BSDL truncated protein and (iii) detecting the presence of said truncated BSDL protein in said sample.

10. The method of claim 9, further comprising the steps of administrating a treatment of pancreatic cancer when a truncated BSDL protein is present.

11. The method of any of the preceding claims, wherein the patient sample is from blood, blood serum, pancreatic juice or pancreatic biopsy, lymph, urine or stools.

12. Use of an antibody which specifically recognizes the PRAAHG Ct-extremity of a BSDL truncated protein for the *in vitro* prognosis and/or diagnosis of a pancreatic cancer.

13. Kit for the prognostic and/or diagnosis of a pancreatic cancer, said kit comprising specific primers allowing the amplification of the VNTR regions of the exon 11 of the *BSDL* gene or the corresponding sequences in mRNA present in a patient sample.

14. Kit for the prognostic and/or diagnosis of a pancreatic cancer, said kit comprising an antibody which specifically recognizes the PRAAHG Ct-extremity of a BSDL truncated protein in a patient sample.

15. Use of a kit of claim 13 or 14 for the prognosis and/or diagnosis of a pancreatic cancer.


SEQUENCE LISTING

<110>  UNIVERSITE D'AIX MARSEILLE
       INSTITUT NATIONAL DE LA SANTE ET DE LA RECHERCHE MEDICALE
       (INSERM)
       CRENON-CONSTARATAS, Isabelle
       MARTINEZ, Emmanuelle
       SILVY, Françoise
       MAS, Eric
       OUAISSI, Mehdi
       LOMBARDO, Dominique

<120>  Diagnosis method of pancreatic cancer

<130>  F2245-19 EP - EMT/11

<160>  7

<170>  PatentIn version 3.5

<210>  1
<211>  21
<212>  DNA
<213>  human

<400>  1
tcttctcact ctgcagggac c                                                21


<210>  2
<211>  22
<212>  DNA
<213>  human

<400>  2
caggggtatg aggctttatt ca                                               22


<210>  3
<211>  20
<212>  DNA
<213>  human

<400>  3
gaaccaactt cctgcgctac                                                  20


<210>  4
<211>  2386
<212>  DNA
<213>  human

<400>  4
ggccacccag aggctgatgc tcaccatggg gcgcctgcaa ctggttgtgt tgggcctcac      60

ctgctgctgg gcagtggcga gtgccgcgaa gctgggcgcc gtgtacacag aaggtgggtt     120

cgtggaaggc gtcaataaga agctcggcct cctgggtgac tctgtggaca tcttcaaggg     180

catccccttc gcagctccca ccaaggccct ggaaaatcct cagccacatc ctggctggca     240

agggaccctg aaggccaaga acttcaagaa gagatgcctg caggccacca tcacccagga     300

cagcacctac ggggatgaag actgcctgta cctcaacatt tgggtgcccc agggcaggaa 360

gcaagtctcc cgggacctgc ccgttatgat ctggatctat ggaggcgcct tcctcatggg 420

gtccggccat ggggccaact tcctcaacaa ctacctgtat gacggcgagg agatcgccac 480

acgcggaaac gtcatcgtgg tcaccttcaa ctaccgtgtc ggcccccttg ggttcctcag 540

cactggggac gccaatctgc caggtaacta tggccttcgg gatcagcaca tggccattgc 600

ttgggtgaag aggaatatcg cggccttcgg gggggacccc aacaacatca cgctcttcgg 660

ggagtctgct ggaggtgcca gcgtctctct gcagaccctc tccccctaca acaagggcct 720

catccggcga gccatcagcc agagcggcgt ggccctgagt ccctgggtca tccagaaaaa 780

cccactcttc tgggccaaaa aggtggctga gaaggtgggt tgccctgtgg gtgatgccgc 840

caggatggcc cagtgtctga aggttactga tccccgagcc ctgacgctgg cctataaggt 900

gccgctggca ggcctggagt accccatgct gcactatgtg ggcttcgtcc ctgtcattga 960

tggagacttc atccccgctg acccgatcaa cctgtacgcc aacgccgccg acatcgacta 1020

tatagcaggc accaacaaca tggacggcca catcttcgcc agcatcgaca tgcctgccat 1080

caacaagggc aacaagaaag tcacggagga ggacttctac aagctggtca gtgagttcac 1140

aatcaccaag gggctcagag gcgccaagac gacctttgat gtctacaccg agtcctgggc 1200

ccaggaccca tcccaggaga ataagaagaa gactgtggtg gactttgaga ccgatgtcct 1260

cttcctggtg cccaccgaga ttgccctagc ccagcacaga gccaatgcca agagtgccaa 1320

gacctacgcc tacctgtttt cccatccctc tcggatgccc gtctacccca aatgggtggg 1380

ggccgaccat gcagatgaca ttcagtacgt tttcgggaag cccttcgcca cccccacggg 1440

ctaccggccc caagacagga cagtctctaa ggccatgatc gcctactgga ccaactttgc 1500

caaaacaggg gaccccaaca tgggcgactc ggctgtgccc acacactggg aaccctacac 1560

tacggaaaac agcggctacc tggagatcac caagaagatg ggcagcagct ccatgaagcg 1620

gagcctgaga accaacttcc tgcgctactg gaccctcacc tatctggcgc tgcccacagt 1680

gaccgaccag gaggccaccc ctgtgccccc cacaggggac tccgaggcca ctcccgtgcc 1740

ccccacgggt gactccgaga ccgcccccgt gccgcccacg ggtgactccg ggcccccccc 1800

cgtgccgccc acgggtgact ccgggccccc ccccgtgccg cccacgggtg actccgggcc 1860

cccccccgtg ccgcccacgg gtgactccgg ggcccccccc gtgccgccca cgggtgactc 1920

cgggcccccc cccgtgccgc ccacgggtga ctccgggccc ccccccgtgc cgcccacggg 1980

tgactccggg gccccccccg tgccgcccac gggtgactcc ggcgcccccc ccgtgccgcc 2040

cacgggtgac gccgggcccc cccccgtgcc gcccacgggt gactccggcg ccccccccgt 2100

gccgcccacg ggtgactccg ggcccccccc cgtgaccccc acgggtgact ccgagaccgc 2160

ccccgtgccg cccacgggtg actccggggc cccccctgtg ccccccacgg gtgactctga 2220

ggctgcccct gtgcccccca cagatgactc caaggaagct cagatgcctg cagtcattag 2280

gttttagcgt cccatgagcc ttggtatcaa gaggccacaa gagtgggacc ccaggggctc 2340

ccctcccatc ttgagctctt cctgaataaa gcctcatacc cctgtc 2386

<210> 5
<211> 756
<212> PRT
<213> human

<400> 5

Met Leu Thr Met Gly Arg Leu Gln Leu Val Val Leu Gly Leu Thr Cys
1               5                   10                  15

Cys Trp Ala Val Ala Ser Ala Ala Lys Leu Gly Ala Val Tyr Thr Glu
            20                  25                  30

Gly Gly Phe Val Glu Gly Val Asn Lys Lys Leu Gly Leu Leu Gly Asp
        35                  40                  45

Ser Val Asp Ile Phe Lys Gly Ile Pro Phe Ala Ala Pro Thr Lys Ala
    50                  55                  60

Leu Glu Asn Pro Gln Pro His Pro Gly Trp Gln Gly Thr Leu Lys Ala
65                  70                  75                  80

Lys Asn Phe Lys Lys Arg Cys Leu Gln Ala Thr Ile Thr Gln Asp Ser
                85                  90                  95

Thr Tyr Gly Asp Glu Asp Cys Leu Tyr Leu Asn Ile Trp Val Pro Gln
            100                 105                 110

Gly Arg Lys Gln Val Ser Arg Asp Leu Pro Val Met Ile Trp Ile Tyr
        115                 120                 125

Gly Gly Ala Phe Leu Met Gly Ser Gly His Gly Ala Asn Phe Leu Asn
    130                 135                 140

Asn Tyr Leu Tyr Asp Gly Glu Glu Ile Ala Thr Arg Gly Asn Val Ile
145                 150                 155                 160

Val Val Thr Phe Asn Tyr Arg Val Gly Pro Leu Gly Phe Leu Ser Thr
                165                 170                 175

Gly Asp Ala Asn Leu Pro Gly Asn Tyr Gly Leu Arg Asp Gln His Met
            180                 185                 190

Ala Ile Ala Trp Val Lys Arg Asn Ile Ala Ala Phe Gly Gly Asp Pro
        195                 200                 205

Asn Asn Ile Thr Leu Phe Gly Glu Ser Ala Gly Gly Ala Ser Val Ser
    210                 215                 220

Leu Gln Thr Leu Ser Pro Tyr Asn Lys Gly Leu Ile Arg Arg Ala Ile
225                 230                 235                 240

Ser Gln Ser Gly Val Ala Leu Ser Pro Trp Val Ile Gln Lys Asn Pro
                245                 250                 255

Leu Phe Trp Ala Lys Lys Val Ala Glu Lys Val Gly Cys Pro Val Gly
            260                 265                 270

Asp Ala Ala Arg Met Ala Gln Cys Leu Lys Val Thr Asp Pro Arg Ala
        275                 280                 285

Leu Thr Leu Ala Tyr Lys Val Pro Leu Ala Gly Leu Glu Tyr Pro Met
    290                 295                 300

Leu His Tyr Val Gly Phe Val Pro Val Ile Asp Gly Asp Phe Ile Pro
305                 310                 315                 320

Ala Asp Pro Ile Asn Leu Tyr Ala Asn Ala Ala Asp Ile Asp Tyr Ile
                325                 330                 335

Ala Gly Thr Asn Asn Met Asp Gly His Ile Phe Ala Ser Ile Asp Met
            340                 345                 350

Pro Ala Ile Asn Lys Gly Asn Lys Lys Val Thr Glu Glu Asp Phe Tyr
        355                 360                 365

Lys Leu Val Ser Glu Phe Thr Ile Thr Lys Gly Leu Arg Gly Ala Lys
    370                 375                 380

Thr Thr Phe Asp Val Tyr Thr Glu Ser Trp Ala Gln Asp Pro Ser Gln
385                 390                 395                 400

Glu Asn Lys Lys Lys Thr Val Val Asp Phe Glu Thr Asp Val Leu Phe
                405                 410                 415

Leu Val Pro Thr Glu Ile Ala Leu Ala Gln His Arg Ala Asn Ala Lys
            420                 425                 430

Ser Ala Lys Thr Tyr Ala Tyr Leu Phe Ser His Pro Ser Arg Met Pro
        435                 440                 445

Val Tyr Pro Lys Trp Val Gly Ala Asp His Ala Asp Asp Ile Gln Tyr
    450                 455                 460

Val Phe Gly Lys Pro Phe Ala Thr Pro Thr Gly Tyr Arg Pro Gln Asp
465                 470                 475                 480

Arg Thr Val Ser Lys Ala Met Ile Ala Tyr Trp Thr Asn Phe Ala Lys
                485                 490                 495

Thr Gly Asp Pro Asn Met Gly Asp Ser Ala Val Pro Thr His Trp Glu
            500                 505                 510

Pro Tyr Thr Thr Glu Asn Ser Gly Tyr Leu Glu Ile Thr Lys Lys Met
        515                 520                 525

Gly Ser Ser Ser Met Lys Arg Ser Leu Arg Thr Asn Phe Leu Arg Tyr
    530                 535                 540

Trp Thr Leu Thr Tyr Leu Ala Leu Pro Thr Val Thr Asp Gln Glu Ala
545                 550                 555                 560

Thr Pro Val Pro Pro Thr Gly Asp Ser Glu Ala Thr Pro Val Pro Pro
                565                 570                 575

Thr Gly Asp Ser Glu Thr Ala Pro Val Pro Pro Thr Gly Asp Ser Gly
            580                 585                 590

Ala Pro Pro Val Pro Pro Thr Gly Asp Ser Gly Ala Pro Pro Val Pro
        595                 600                 605

Pro Thr Gly Asp Ser Gly Ala Pro Pro Val Pro Pro Thr Gly Asp Ser
    610                 615                 620

Gly Ala Pro Pro Val Pro Pro Thr Gly Asp Ser Gly Ala Pro Pro Val
625                 630                 635                 640

Pro Pro Thr Gly Asp Ser Gly Ala Pro Pro Val Pro Pro Thr Gly Asp
                645                 650                 655

Ser Gly Ala Pro Pro Val Pro Pro Thr Gly Asp Ser Gly Ala Pro Pro
            660                 665                 670

Val Pro Pro Thr Gly Asp Ala Gly Pro Pro Pro Val Pro Pro Thr Gly
        675                 680                 685

Asp Ser Gly Ala Pro Pro Val Pro Pro Thr Gly Asp Ser Gly Ala Pro
    690                 695                 700

Pro Val Thr Pro Thr Gly Asp Ser Glu Thr Ala Pro Val Pro Pro Thr
705                 710                 715                 720

Gly Asp Ser Gly Ala Pro Pro Val Pro Pro Thr Gly Asp Ser Glu Ala
                725                 730                 735

Ala Pro Val Pro Pro Thr Asp Asp Ser Lys Glu Ala Gln Met Pro Ala
            740                 745                 750

Val Ile Arg Phe
        755

<210>  6
<211>  9
<212>  PRT
<213>  human

<400>  6

Gly Ala Pro Pro Arg Ala Ala His Gly
1               5

<210>  7
<211>  12
<212>  PRT
<213>  human

<400>  7

Cys Lys Glu Ala Gln Met Pro Ala Val Ile Arg Phe
1               5                   10